# EUROPÄISCHE PATENTSCHRIFT

(11) **EP 1 694 222 B1**
(45) Veröffentlichungstag und Bekanntmachung des Hinweises auf die Patenterteilung: **22.07.2015**
(21) Anmeldenummer: 04738694.1
(22) Anmeldetag: 18.06.2004
(51) Int. Cl.: A61B 17/32, A61B 17/22

(54) **WASSERSTRAHLEINRICHTUNG ZUM TRENNEN EINER BIOLOGISCHEN STRUKTUR**
WATER-JET DEVICE FOR SEPARATING A BIOLOGICAL STRUCTURE
DISPOSITIF A JET D'EAU DESTINE A SEPARER UNE STRUCTURE BIOLOGIQUE

(30) Priorität: 20.06.2003 DE 20309616 U
(43) Veröffentlichungstag der Anmeldung: 30.08.2006
(73) Patentinhaber: Human Med AG, 19061 Schwerin (DE)
(72) Erfinder: Pein, Andreas, 23911 Einhaus (DE)
(74) Vertreter: Schneider, Henry
(86) Internationale Anmeldenummer: PCT/DE2004/001243
(87) Internationale Veröffentlichungsnummer: WO 2004/112623

(56) Entgegenhaltungen:
- US-A- 5 370 609
- US-A- 5 931 647
- US-A1- 2001 043 874
- US-B1- 6 216 573

## Beschreibung

Die Erfindung bezieht sich auf eine Wasserstrahleinrichtung nach dem Oberbegriff des Anspruchs 1.

Derartige Wasserstrahleinrichtungen werden insbesondere in der Humanmedizin eingesetzt.

Eine solche Wasserstrahleinrichtung ist in der EP 0 551 920 B1 beschrieben. Diese Wasserstrahleinrichtung besteht im wesentlichen aus einem Druckerzeuger, einer Kolben-Zylinder-Einheit und einer Trenneinrichtung in Form eines speziellen Operationshandstückes. Im Zylinderraum der Kolben-Zylinder-Einheit ist formschlüssig eine Kartusche eingesetzt, die mit einer sterilen Trennflüssigkeit gefüllt ist. Diese Kartusche hat einerseits Kontakt mit dem Kolben der Kolben-Zylinder-Einheit und ist andererseits über eine Druckleitung mit dem Operationshandstück verbunden. Im Betrieb belastet das Druckmedium des Druckerzeugers die Kolben-Zylinder-Einheit und damit die Kartusche, in dessen Folge die Trennflüssigkeit aus der Kartusche zum Operationshandstück gefördert wird. Dort tritt die Trennflüssigkeit in Form eines feinen Flüssigkeitsstrahles aus. Durch die Trennung des Trennflüssigkeitskreislaufes von dem Druckflüssigkeitskreislauf und durch eine besondere Ausführung aller Geräteeinheiten des Trennflüssigkeitskreislaufes wird eine durchgehende Sterilkette gewährleistet.

Es hat sich aber gezeigt, dass eine solche Wasserstrahleinrichtung wegen eines begrenzten Füllungsvolumens der Kartusche für solche Anwendungsfälle ungeeignet ist, die eine größere Trennflüssigkeitsmenge erfordern. Dazu gehören alle Anwendungen der plastischen Chirurgie, insbesondere dem Fettabsaugen. Ein weiterer Nachteil besteht darin, dass der gleichmäßig austretende Flüssigkeitsstrahl für besondere Anwendungsfälle mit einem zusätzlichen Aggregat gepulst werden muss. Dieser Aufwand ist relativ groß.

Aus der US 6.216.573 B1 wurde nun eine Wasserstrahleinrichtung bekannt, die diese Nachteile nicht hat. Diese Wasserstrahleinrichtung besteht aus einem höhergelegenen Vorratsbehälter für die Trennflüssigkeit, einer Kolbenpumpe und einem Operationshandstück. Der höhergelegene Vorratsbehälter ist über eine Fallleitung mit der Kolbenpumpe verbunden und von der Kolbenpumpe führt eine Druckleitung zum Operationshandstück. Dazu besteht die Kolbenpumpe aus einem Pumpengehäuse mit der Kolbenzylindereinheit und einem den Kolben der Kolben-Zylinder-Einheit antreibenden Exzenterantrieb, wobei in den Zylinderraum der Kolben-Zylinder-Einheit eine membrane Flüssigkeitsblase eingesetzt ist und der restliche Zylinderraum mit einer Druckflüssigkeit befüllt ist. Die membrane Flüssigkeitsblase ist einerseits über die Fallleitung mit dem Vorratsbehälter und andererseits über ein öffnendes Rückschlagventil und über die Druckleitung mit dem Operationshandstück verbunden.

Die membrane Flüssigkeitsblase wird durch die Bewegung des Kolbens allseitig und wechselweise mit einem Überdruck oder Unterdruck beaufschlagt, sodass die im Vorratsbehälter befindliche Trennflüssigkeit pulsartig angesaugt und anschließend pulsartig zum Operationshandstück befördert wird.

Auch diese Wasserstrahleinrichtung hat Nachteile. So erzeugt die Kolben-Zylinder-Einheit keinen definierten und scharfen Trennstrahl, weil der Trennstrahl in der Hauptsache von der Volumenveränderung der Flüssigkeitsblase bestimmt wird und diese Volumenveränderung ist nicht reproduzierbar, weil die Flüssigkeitsblase lediglich von zwei Flüssigkeitssäulen eingespannt ist. Die Kolben-Zylinder-Einheit ist auch nicht leistungsfähig genug. Das hängt in erster Linie damit zusammen, dass die Richtung der in die Flüssigkeitsblase einfallenden Flüssigkeitsstrom und die Richtung des aus der Flüssigkeitsblase austretenden Druckflüssigkeitsstromes entgegengerichtet sind. Das führt zu einer Umkehr der Flüssigkeitsströmung innerhalb der Flüssigkeitsblase und damit zu Turbulenzen und zu einem Druckabfall.

Nachteilig ist auch, dass es wieder zwei getrennte Flüssigkeitskreisläufe gibt, die wegen der Forderung nach Sterilität unter hohem Aufwand voneinander zu trennen sind.

Ein wesentlicher Nachteil besteht aber darin, dass die Kolben-Zylinder-Einheit und der Exzenterantrich in einem gemeinsamen Pumpengehäuse untergebracht sind, das dadurch groß- und schwerbauend wird und nur stationär eingesetzt werden kann. Das verteuert die Herstellung und schränkt den Einsatzbereich stark ein, da das Volumen der Flüssigkeitsblase nur auf einen ausgewählten Anwendungsbereich ausgelegt ist. Eine Anpassung der Kolben-Zylinder-Einheit an andere Anwendungställe mit einem größercn Trennflüssigkeitsbedarf ist wegen der Kompaktheit des Pumpengehäuses nicht möglich und der Einsatz einer größeren Flüssigkeitsblase im vorhanden Pumpengehäuse scheidet aus, weil die Flüssigkeitsblase und die Kolben-Zylinder-Einheit zueinander passen müssen.

In der US 5,370,609 A ist nun eine Wasserstrahleinrichtung gemäß dem Oberbegriff des Anspruchs 1 zur Entfernung von Thrombusablagerungen beschrieben, die im Wesentlichen aus einem Vorratsbehälter mit einer Trennflüssigkeit, aus einer angetriebenen Kolben-Zylinder-Einheit zum Aufbau eines Trennstrahles, aus einem Operationshandstück und aus einer Saugeinrichtung zur Absaugung der abgetrennten Thrombusablagerungen besteht. Dabei ist die KolbenZylinder-Einheit über eine Saugleitung mit dem Vorratsbehälter und über eine Druckleitung und einer Verteilervorrichtung mit dem Operationshandstück verbunden. Über die gleiche Verteilervorrichtung besteht auch eine Verbindung zwischen dem Operationshandstück und der Saugeinrichtung.

Die Kolben-Zylinder-Einheit ist aus hygienischen Gründen wegwerfbar und damit von allen Baugruppen trennbar ausgeführt, während alle anderen Baugruppen unlösbar miteinander verbunden sind.

Das hat Nachteile. So müssen auch der Verteiler und das Operationshandstück aus hygienischen Gründen nach jedem Einsatz ausgetauscht werden, was bedeutet, dass dann gleichzeitig auch alle anderen, hygienisch nicht so sensiblen Baugruppen, wie beispielsweise die Saugeinrichtung weggeworfen werden müssen. Außerdem führt die Kompaktheit der Wasserstrahleinrichtung dazu, dass sie nur für spezielle Anwendungsfälle verwendet werden kann. Das schränkt den Einsatzbereich stark ein. Für unterschiedliche Anwendungsfälle muss dann immer eine spezielle Wasserstrahleinrichtung in ihrer gesamten Kompaktheit vorgesehen werden. Das verteuert die Anwendung der Wasserstrahleinrichtung.

Der Erfindung liegt daher die Aufgabe zu Grunde, eine gattungsgemäße Wasserstrahleinrichtung zum Trennen einer biologischen Struktur zu entwickeln, die nach dem Baukastenprinzip aufgebaut ist und die durch Austausch einzelner Geräte an unterschiedlichen Anwendungställe angepasst werden kann.

Diese Aufgabe wird durch die kennzeichnenden Merkmale des Anspruches 1 gelöst. Weitere Ausgestaltungsmöglichkeiten ergeben sich aus den Unteransprüchen 2 bis 7. Die neue Wasserstrahleinrichtung beseitigt die genannten Nachteile des Standes der Technik.

Der besondere Vorteil der neuen Wasserstrahleinrichtung liegt darin, dass die Kolben-Zylinder-Einheit als eine einzelne Baugruppe ausgeführt ist und damit unabhängig von der exzentrischen Antriebseinrichtung wird. Dadurch wird es möglich, die Kolben-Zylinder-Einheit einfach und kostengünstig herzustellen und sie mit den dazugehörenden Druck- und Saugleitungen und mit dem Operationshandstück zu einem Set zusam leitung in den Zwischenraum zwischen der Presshülse und der Druckkanüle geschoben und zusammen radial verpresst.

Eine einfache Montage des Druckanschlusses ergibt sich, wenn der Sauganschluss und der Druckanschluss radial gegenüberliegen. Dann kann die Druckkanüle über den Sauganschluss von innen in das Zylindergehäuse eingeschoben und verpresst werden.

Die radial gegenüberliegende Anordnung von Sauganschluss und Druckanschluss hat außerdem den großen Vorteil, dass der eintretende Saugstrom und der austretende Druckstrom gleichgerichtet sind. Das vermeidet unnötige Druckverluste in der Strömung und damit einen hohen energetischen Wirkungsgrad.

Die Erfindung soll anhand eines Ausführungsbeispieles näher erläutert werden.

Dazu zeigen:

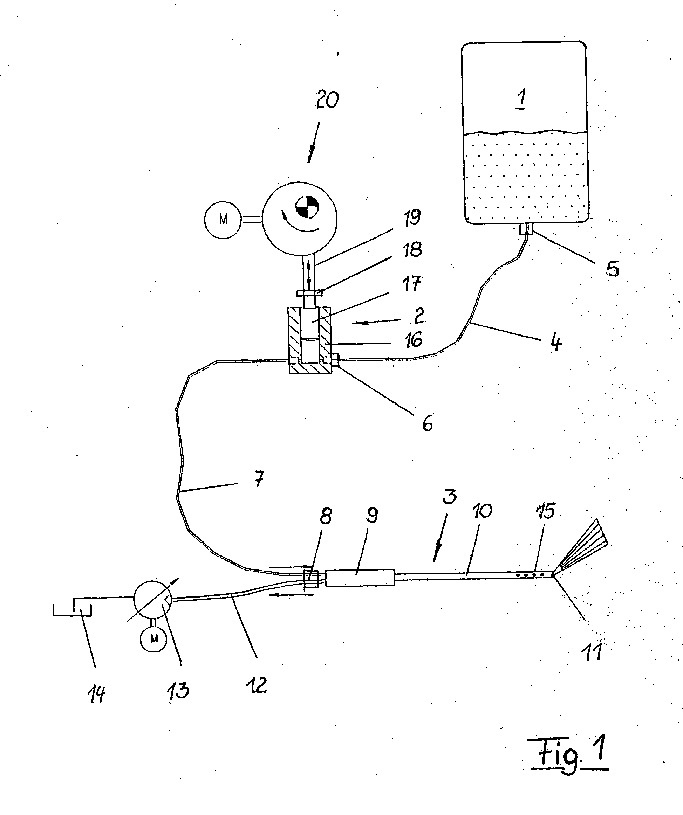

Fig. 1: eine schematische Darstellung der Wasserstrahleinrichtung und

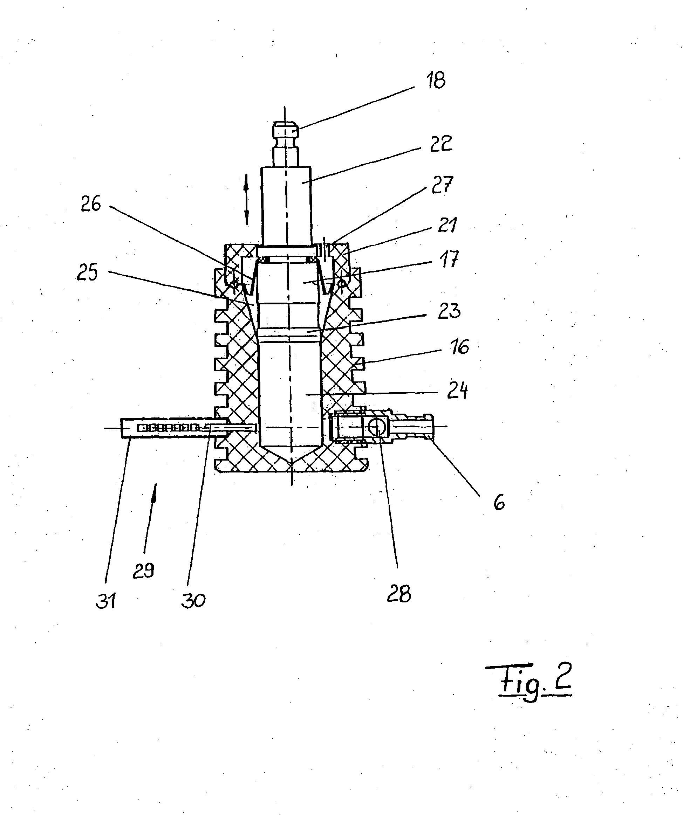

Fig. 2: eine Kolbenpumpe der Wasserstrahleinrichtung.

Nach der Fig, 1 besteht die Wasserstrahleinrichtung zum Trennen einer biologischen Struktur aus einem Vorratsbehälter 1 für die zu verwendende Trennflüssigkeit, einer Kolben-Zylinder-Einheit 2 und einem Operationshandstück 3. Dabei ist der Vorratsbehälter 1 und die Kolben-Zylinder-Einheit 2 über eine Saugleitung 4 miteinander verbunden, wobei die Saugleitung 4 über eine erste Steckkupplung 5 mit dem Vorratsbehälter 1 und über eine zweite Steckkupplung 6 mit der Kolben-Zylinder-Einheit 2 verbunden ist. Dagegen ist die Kolben-Zylinder-Einheit 2 mit dem Operationshandstück 3 über eine Druckleitung 7 verbunden, wobei die Verbindung mit der Kolben-Zylinder-Einheit 2 über eine vorzugsweise nicht lösbare Verbindung und die Verbindung mit dem Operationshandstück 3 über eine dritte Steckkupplung 8 realisiert wird.

Das Operationshandstück 3 besteht in bekannter Weise aus einem Handstück 9 und einem Druck- und Saugrohr 10. Das Druck- und Saugrohr 10 besitzt eine innen liegende Druckkanüle mit einer am Ende befindlichen Austrittsdüse 11, die mit der zum Operationshandstück 3 führenden Druckleitung 7 in Verbindung steht, und es besitzt ein Saugrohr, das die Druckkanüle unter Bildung eines Ringkanals ummantelt und das über eine Absaugleitung 12 und eine angetriebene Absaugpumpe 13 mit einem Aufnahmetank 14 verbunden ist. Das Saugrohr besitzt weiterhin an seinem Umfang verteilt angeordnete radiale Absaugöffnungen 15 zur Aufnahme der abgetrennten Gewebeteilchen und der angesammelten Trennflüssigkeit.

Die Kolben-Zylinder-Einheit 2 besteht aus einem Zylindergehäuse 16 und einem im Zylindergehäuse 16 mit Spiel eingepassten Kolben 17. Dieser Kolben 17 ist über eine vierte Steckkupplung 18 mit dem Betätigungsstößel 19 einer exzentrischen Antriebseinrichtung 20 verbunden.

Die Kolben-Zylinder-Einheit 2 ist in der Fig, 2 näher dargestellt und zeigt wieder das Zylindergehäuse 16 und den Kolben 17 mit seiner Steckkupplung 18 für die exzentrische Antriebseinrichtung 20. Das Zylindergehäuse 16 besitzt eine Grundbohrung mit einem zylindrischen Teil, der dem verschlossenen Ende der Grundbohrung nahe liegt, und aus einem konischen Teil, der im Bereich des offenen Endes der Grundbohrung liegt. Dabei ist das offene Ende der Grundbohrung durch einen Gehäusedeckel 21 druckdicht verschlossen. In die Grundbohrung des Zylindergehäuses 16 ist der Kolben 17 eingesetzt, der mit einem Kupplungsschaft 22 den Zylinderdeckel 21 durchdringt und andererseits eine Dichtlippe 23 besitzt. Dabei bilden der Kolben 17 mit seiner Dichtlippe 23 und die Grundbohrung des Zylindergehäuses 16 mit ihren zylindrischen Teil einen Saug- und Druckraum 24, während sich zwischen dem Umfang des Kolbens 17 und dem konischen Teil der Grundbohrung ein ringförmiger Parkstellungsraum 25 ergibt. Dabei sind die Längenverhältnisse zwischen dem Kolben 17 und den beiden Teilen der Grundbohrung des Zylindergehäuses 16 so gewählt, dass sich der Kolben 17 in seiner ausgefahrenen Endstellung im Anschlag mit dem Gehäusedeckel 21 befindet und die Dichtlippe 23 dabei eine Position im Übergangsbereich vom zylindrischen zum konischen Teil der Grundbohrung einnimmt. In dieser Position ist die Dichtlippe 23 bereits etwas entspannt, gewährleistet aber noch eine ausreichende Dichtheit zwischen dem Saug- und Druckraum 24 und dem Parkstellungsraum 25. Zwischen dem Kolben 17 und dem Zylindergehäuse 16 ist eine Membran 26 angeordnet, die einerseits zwischen dem Zylindergehäuse 16 und dem Gehäusedeckel 21 eingespannt ist und andererseits in eine Ringnut des Kolbens 17 befestigt ist. Diese Membran 26 hat dabei im Zusammenwirken mit dem Parkstellungsraum 25 eine solche Bewegungsfreiheit, dass ein ausreichender und hemmungsfreier Hub des Kolbens 17 gewährleistet wird. Der Gehäusedeckel 21 ist weiterhin mit einer Luftausgleichsbohrung 27 ausgestattet, die den volumenveränderlichen Luftraum zwischen der Membran 26 und dem Gehäusedeckel 21 mit der Atmosphäre verbindet.

Der Saug- und Druckraum 24 besitzt einerseits einen radialen Sauganschluss, der über die zweite Steckkupplung 6 mit der zum Vorratsbehälter 1 führenden Saugleitung 4 verbunden ist. In diesen Sauganschluss ist ein in Saugrichtung öffnendes Rückschlagventil 28 eingesetzt. Gegenüberliegend zum Sauganschluss befindet sich ein radialer Druckanschluss 29. Dieser Druckanschluss 29 besteht aus einer Druckkanüle 30, die von innen durch den Sauganschluss in das Zylindergehäuse 16 eingepresst ist und die an ihrem herausragenden Ende mit äußeren Pressrippen ausgestattet ist. Über diese Druckkanüle 30 ist von außen eine Presshülse 31 in das Zylindergehäuse 16 eingepresst, die einen Ringspalt zur Druckkanüle 30 freihält. In diesen Ringspalt ist die zum Operationshandstück 3 führende Druckleitung 7 eingeschoben und unter einer auf die Presshülse 31 wirkenden Kraft mit der Druckkanüle 30 verpresst.

Im Betrieb versetzt die exzentrische Antriebseinrichtung 20 den Kolben 17 der Kolben-Zylinder-Einheit 2 in eine pendelnde Bewegung, bei welcher der Kolben 17 wechselweise ein- und ausfährt. Beim Ausfahren des Kolbens 17 vergrößert sich der Saug- und Druckraum 24, sodass ein Unterdruck entsteht, der das Rückschlagventil 28 in der Saugleitung 4 öffnet und die Trennflüssigkeit aus dem Vorratsbehälter 1 ansaugen lässt. Dieser Ansaugvorgang wird dadurch unterstützt, dass der Vorratsbehälter in einer höherliegenden Position gebracht ist und somit der potentielle Druck der im Vorratsbehälter 1 befindlichen Trennflüssigkeit genutzt wird. Beim Einfahren des Kolbens 17 verkleinert sich der Saug- und Druckraum 24 und es entsteht ein Überdruck, der das in der Saugleitung 4 befindliche Rückschlagventil 28 schließt und die im Saug- und Druckraum 24 befindliche Trennflüssigkeit über die Druckkanüle 30 in die Druckleitung 7 befördern lässt. Von dort gelangt die Trennflüssigkeit in bekannter Weise zum Operationshandstück 3, wo sie aus der Austrittsdüse 11 als ein gebündelter oder gefächerter Trennstrahl austritt. Die ausgetretene Trennflüssigkeit und die abgetrennten Gewebeteilchen werden im gleichen Zuge durch die Wirkung der Absaugpumpe 13 abgesaugt und in einen Aufnahmetank 14 abgelegt.

### Liste der Bezugszeichen

- 1: Vorratsbehälter
- 2: Kolben- Zylinder-Einheit
- 3: Operationshandstück
- 4: Saugleitung
- 5: erste Steckkupplung
- 6: zweite Steckkupplung
- 7: Druckleitung
- 8: dritte Steckkupplung
- 9: Handstück
- 10: Druck- und Saugrohr
- 11: Austrittsdüse
- 12: Absaugleitung
- 13: Absaugpumpe
- 14: Aufnahmetank
- 15: Absaugöffnung
- 16: Zylindergehäuse
- 17: Kolben
- 18: vierte Steckkupplung
- 19: Betätigungsstößel
- 20: Exzentrische Antriebseinrichtung
- 21: Gehäusedeckel
- 22: Kupplungsschaft
- 23: Dichtlippe
- 24: Saug- und Druckraum
- 25: Parkstellungsraum
- 26: Membran
- 27: Luftaustrittsöffnung
- 28: Rückschlagventil
- 29: Druckanschluss
- 30: Druckkanüle
- 31: Presshülse

## Patentansprüche

1. Wasserstrahleinrichtung zum Trennen einer biologischen Struktur, bestehend aus einem Vorratsbehälter (1) mit einer Trennflüssigkeit, aus einer Kolben-Zylinder-Einheit (2) mit einer exzentrischen Antriebseinrichtung (20) und aus einem OperaUonshandstück (3), wobei die Kolben-Zylinder-Einheit (2) über eine Saugleitung (4) mit dem Vorratsbehälter (1) und über eine Druckleitung (7) mit dem Operationshandstück (3) verbunden ist, aus einem Zylindergehäuse (16) und einem im Zylindergehäuse (16) eingepassten und mit einer exzentrischen Antriebseinrichtung (20) verbundenen Kolben (17) besteht, die beide einen Saug- und Druckraum (24) ausbilden, und mit der exzentrischen Antrichseinrichtung (20) trennbar verbunden ist,
**dadurch gekennzeichnet, dass** die Kolben-Zylinder-Einheit (2) mit der Saugleitung (4), der Druckleitung (7) und dem Operationshandstück (3) als ein anwendungsspezifisches Applikations-Set ausgebildet ist, der Saug- und Druckraum (24) der Kolben-Zylinder-Einheit (2) nach außen durch eine Membran (26) abgedichtet ist und das Zylindergehäuse (16) und der Kolben (17) neben dem Saug- und Druckraum (24) einen ringförmigen Parkstellungsraum (25) ausbilden, in dem einerseits das Dichtelement des Kolbens (17) in der ausgefahrenen Endstellung des Kolbens (17) eintaucht und entspannt und in dem andererseits die Membran (26) einen ausreichenden Bewegungsfreiraum findet.

2. Wasserstrahleinrichtung nach Anspruch 1,
**dadurch gekennzeichnet, dass** der Parkstellungsraum (25) zumindest im Übergangsbereich zum Saug- und Druckraum (24) konisch aufgeführt ist und das Dichtelement des Kolbens (17) durch den Konus des Parkstellungsraumes (5) entspannt wird.

3. Wasserstrahleinrichtung nach Anspruch 2,
**dadurch gekennzeichnet, dass** das Zylindergehäuse (16) und der Kolben der Kolben-Zylinder-Einheit (2) aus Kunststoff besteht und das Dichtelement des Kolbens als eine an den Kolben (17) angearbeitete und überstehende Dichtlippe (23) ausgeführt ist.

4. Wasserstrahleinrichtung nach Anspruch 1,
**dadurch gekennzeichnet, dass** der Druckanschluss (29) des Saug- und Druckraumes (24) als eine Pressverbindung ausgeführt ist, bei der jeweils eine Druckkanüle (30) mit überstehenden Pressrippen und eine Presshülse (31) in das Zylindergehäuse (16) eingepresst sind und die Presshulse (31) die Pressrippen der Druckkanüle (30) in einem solchen Abstand umgreift, der der Materialstärke der Druckleitung (7) entspricht.

5. Wasserstrahleinrichtung nach Anspruch 4,
**dadurch gekennzeichnet, dass** der Sauganschluss und der Druckanschluss (29) radial zum Zylindergehäuse (16) und so gegenüberliegend zueinander angeordnet sind, dass die Druckkanüle (30) von innen durch den Sauganschluss und dem Saug- und Druckraum (16) montiert werden kann.

## Claims

1. A water-jet device for separating a biological structure, comprising a storage container (1) with a separating fluid, a piston cylinder unit (2) having an eccentric drive device (20), and an operating handpiece (3), wherein the piston cylinder unit (2) is connected via a suction line (4) to the storage container (1) and via a pressure line (7) to the operating handpiece (3), a cylinder casing (16) and a piston (17) fitted in the cylinder casing (16) and connected to an eccentric drive device (20), which both form a suction and pressure space (24), and is connected to the eccentric drive device (20) in a separable manner,
**characterized in that** the piston cylinder unit (2) is embodied as an applicationspecific application set with the suction line (4), the pressure line (7) and the operating handpiece (3), the suction and pressure space (24) of the piston cylinder unit (2) is sealed externally by a membrane (26) and the cylinder casing (16) and the piston (17), in addition to the suction and pressure space (24), form an annular fixing region (25) in which, on the one hand, the sealing element of the piston (17) submerges and loses tension in the extended final position of the piston (17) and in which, on the other hand, the membrane (26) has sufficient free space to move.

2. The water-jet device according to Claim 1,
**characterized in that** the fixing region (25) is designed conically at least in the transition area with the suction and pressure space (24) and the sealing element of the piston (17) is released by the conical portion of the fixing region (25).

3. The water-jet device according to Claim 2,
**characterized in that** the cylinder casing (16) and the piston of the piston cylinder unit (2) are made of plastic and the sealing element of the piston is designed as a projecting sealing lip (23) machined on to the piston (17).

4. The water-jet device according to Claim 1,
**characterized in that** the pressure intake (29) of the suction and pressure space (24) is designed as a press connection, **wherein** in each case a pressure tubule (30) having projecting press ribs and a press sleeve (31) are pressed into the cylinder casing (16) and the press sleeve (31) encircles the press ribs of the pressure tubule (30) at such a distance that corresponds to the material thickness of the pressure line (7).

5. The water-jet device according to Claim 4,
**characterized in that** the suction intake and the pressure intake (29) are situated radially to the cylinder casing (16) and opposite to each other in such a manner that the pressure tubule (30) can be mounted from inside through the suction intake and the suction and pressure space (16).

## Revendications

1. Dispositif à jet d'eau (1) destiné à séparer une structure biologique, composé d'un réservoir de stockage (1) avec un liquide de séparation, d'une unité piston-cylindre (2) avec un dispositif d'entraînement (20) excentré et d'une pièce à main de manoeuvre (3), l'unité piston-cylindre (2) étant raccordée au réservoir de stockage (1) par le biais d'une conduite d'aspiration (4) et à la pièce à main de manoeuvre (3) par le biais d'une conduite de pression (7), d'un boîtier de cylindre (16) et d'un piston (17) inséré dans le boîtier de cylindre (16) et raccordé à un dispositif d'entraînement (20) excentré qui constituent tous deux une chambre d'aspiration et de pression (24), et qui est raccordé de façon détachable au dispositif d'entraînement (20) excentré,
**caractérisé en ce que** l'unité piston-cylindre (2) avec la conduite d'aspiration (4), la conduite de pression (7) et la pièce à main de manoeuvre (3) est constituée en tant que kit d'application spécifique à l'utilisation, **en ce que** la chambre d'aspiration et de pression (24) de l'unité piston-cylindre (2) est rendue étanche vers l'extérieur par une membrane (26), et **en ce que** le boîtier de cylindre (16) et le piston (17) constituent près de la chambre d'aspiration et de pression (24) un espace de stationnement (25) annulaire dans lequel d'une part l'élément d'étanchéité du piston (17), dans la position extrême sortie du piston (17), plonge et se détend et dans lequel d'autre part la membrane (26) trouve un espace de liberté de mouvement suffisant.

2. Dispositif à jet d'eau selon la revendication 1,
**caractérisé en ce que** l'espace de stationnement (25) est réalisé de façon conique au moins dans la zone de transition avec la chambre d'aspiration et de pression (24) et **en ce que** l'élément d'étanchéité du piston (17) est détendu par le cône de l'espace de stationnement (25).

3. Dispositif à jet d'eau selon la revendication 2,
**caractérisé en ce que** le boîtier de cylindre (16) et le piston de l'unité piston-cylindre (2) sont composés de matière plastique et **en ce que** l'élément d'étanchéité du piston est réalisé ne tant que lèvre d'étanchéité (23) saillante et ajustée sur le piston (17).

4. Dispositif à jet d'eau selon la revendication 1,
**caractérisé en ce que** le raccord de pression (29) de la chambre d'aspiration et de pression (24) est réalisé en tant que raccordement par compression dans lequel respectivement une canule de pression (30) avec des nervures de compression saillantes et une douille de compression (31) sont insérées par pression dans le boîtier de cylindre (16), et la douille de compression (31) entoure les nervures de compression de la canule de pression (30) à une distance qui correspond à l'épaisseur de matériau de la conduite de pression (7).

5. Dispositif à jet d'eau selon la revendication 4,
**caractérisé en ce que** le raccord d'aspiration et le raccord de pression (29) sont **disposés** radialement par rapport au boîtier de cylindre (16) et de façon opposée l'un par rapport à l'autre de telle sorte que la canule de pression (30) peut être montée de l'intérieur à travers le raccord d'aspiration et le boîtier de cylindre (16).
